# EUROPÄISCHE PATENTSCHRIFT

(11) **EP 2 376 420 B1**
(45) Veröffentlichungstag und Bekanntmachung des Hinweises auf die Patenterteilung: **14.11.2018**
(21) Anmeldenummer: 09756526.1
(22) Anmeldetag: 25.11.2009
(51) Int. Cl.: B22F 3/22, C04B 35/632, C04B 35/634, C04B 35/111, C04B 35/465, C04B 35/486, C04B 35/484

(54) **VERWENDUNG EINES ESTERS IN EINEM BINDERSYSTEM**
USE OF AN ESTER IN A BINDER SYSTEM
UTILISATION D'UN ESTER DANS UN SYSTÈME LIANT

(30) Priorität: 15.12.2008 DE 102008054615
(43) Veröffentlichungstag der Anmeldung: 19.10.2011
(73) Patentinhaber: Robert Bosch GmbH, 70442 Stuttgart (DE)
(72) Erfinder: GRATH, Eberhard, 87509 Immenstadt (DE); AICHELE, Wilfried, 71364 Winnenden (DE); RAGER, Jochen, 72406 Bisingen (DE); ARNOLD, Josef, 87509 Immenstadt (DE); CZERWINSKI, Klaus, 71296 Heimsheim (DE); WEISSER, Eva, 71334 Waiblingen (DE)
(86) Internationale Anmeldenummer: PCT/EP2009/065834
(87) Internationale Veröffentlichungsnummer: WO 2010/072503

(56) Entgegenhaltungen:
- DE-A1- 19 942 541
- DE-C2- 4 129 952
- "LOXIOL 2472", , 17. März 2006 (2006-03-17), XP055242286, Gefunden im Internet: URL:http://shtchem.img6.kr/data/LOXIOL2472 .pdf [gefunden am 2016-01-18]
- DATABASE CA [Online] CHEMICAL ABSTRACTS SERVICE, COLUMBUS, OHIO, US; 1968, OZCAN, YILDIZ: "The C1-22 alkyl salicylates" XP002568258 gefunden im STN Database accession no. 1968:476846 & OZCAN, YILDIZ: "The C1-22 alkyl salicylates" ISTANBUL UNIVERSITESI FEN FAKULTESI MECMUASI, SERI C: [ASTRONOMI-FIZIK-KIMYA] , 31(1-4), 89-93 CODEN: IFMCAL; ISSN: 0444-7298, 1966,
- DATABASE CA [Online] CHEMICAL ABSTRACTS SERVICE, COLUMBUS, OHIO, US; 1987, INOUE, TAKESHI ET AL: "Preparation of docosyl p-hydroxybenzoate as a termite repellent" XP002568259 gefunden im STN Database accession no. 1987:439425 & JP 62 072651 A (YOSHITOMI PHARMACEUTICAL INDUSTRIES, LTD., JAPAN) 3. April 1987 (1987-04-03)
- DATABASE CA [Online] CHEMICAL ABSTRACTS SERVICE, COLUMBUS, OHIO, US; 1971, RODIONOV, P. P. ET AL: "Alkyl salicylates" XP002568260 gefunden im STN Database accession no. 1971:488267 & RODIONOV, P. P. ET AL: "Alkyl salicylates" METODY POLUCHENIYA KHIMICHESKIKH REAKTIVOV I PREPARATOV , NO. 18, 236-40 FROM: REF. ZH., KHIM. 1970, ABSTR. NO. 10ZH257 CODEN: MPRPAT; ISSN: 0539-5143, 1969,
- CHIEN-TIEN CHEN, JEN-HUANG KUO, CHENG-HSIU KU, SHIUE-SHIEN WENG, AND CHENG-YUAN LIU: "Nucleophilic Acyl Substitutions of Esters with Protic Nucleophiles Mediated by Amphoteric, Oxotitanium, and Vanadyl Species" JOURNAL OF ORGANIC CHEMISTRY., Bd. 70, Nr. 4, 22. Januar 2005 (2005-01-22), Seiten 1328-1339, XP002568261 AMERICAN CHEMICAL SOCIETY. EASTON., US ISSN: 0022-3263 DOI: 10.1021/jo0484878
- "LOXIOL 2472", , 27 July 2009 (2009-07-27), XP055242286, Retrieved from the Internet: URL:http://shtchem.img6.kr/data/LOXIOL2472 .pdf [retrieved on 2016-01-18]

## Beschreibung

### Stand der Technik

Die Erfindung betrifft die Verwendung eines Esters aus einer Hydroxybenzoesäure und einem Fettalkohol in einem Bindersystem für ein Pulverspritzgussverfahren. Bindersysteme, die im Pulverspritzguss eingesetzt werden, enthalten im Allgemeinen eine polymere Binderkomponente, eine weitere Binderkomponente, einen sogenannten Co-Binder, die mit der polymeren Binderkomponente verträglich ist, und gegebenenfalls einen Dispergator, um Metall- oder Keramikpulverteilchen im geschmolzenen Binder in Dispersion zu halten. Verträglichkeit der weiteren Binderkomponente mit der polymeren Binderkomponente bedeutet, dass die Binderkomponenten in der Schmelze eine einheitliche flüssige Phase bilden und dass auch im festen Zustand vorwiegend eine einheitliche Binderphase (feste Lösung) vorliegt, wobei die weitere Binderkomponente zusätzlich aus der gemeinsamen festen Phase unterhalb deren Schmelzpunkt entfernbar sein muss, beispielsweise durch Extraktion, wobei die polymere Binderkomponente weder in größerem Maße quellen noch Risse bilden darf.

Als polymere Binderkomponente werden häufig Polyamide eingesetzt. Als geeignete Co-Binder für Polyamide sind zum Beispiel aus EP-B 0 809 556 oder aus US 5,002,988 N,N'-Diacetylpiperazin oder aus DE-C 41 29 952 Alkylphenole bekannt. Wenn Alkylphenole eingesetzt werden, so handelt es sich hierbei im Allgemeinen um solche mit C₁-C₂₀-Alkyl.

Insbesondere bei der Verwendung des Gemisches verschiedener Verbindungen, das unter anderem Ester der p-Hydroxybenzoesäure mit verschiedenen Fettalkoholen enthält, hat sich gezeigt, dass die spritzgegossenen Teile zum Ankleben am Spritzgießwerkzeug neigen.

Diese Nachteile sind insbesondere darauf zurückzuführen, dass Fettalkohole aus Naturprodukten gewonnen werden und im Allgemeinen bereits als Gemisch anfallen. Zudem entsteht bei dem bekannten Syntheseverfahren zur Herstellung von Estern aus einer Hydroxybenzoesäure mit einem Fettalkohol Phenol als Nebenprodukt.

Diesbezüglich ist der Publikation Chien-Tien Chen et al "Nucleophilic Acyl Substitutions of Esters with Protic Nucleophiles Mediated by Amphoteric, Oxotitanium, and Vanadyl Species" J. Org. Chem. 2005, 70, 1328-1339 sind Katalysatoren zur nukleophilen Substitution bei Umesterungsreaktionen bekannt.

Weiterhin ist der DE 19942541 A1 ein Verfahren zur Herstellung von Carbonsäureestern zu entnehmen, das unter anderem auf der Umesterung von Carbonsäureestern in Gegenwart von Schwermetall-Katalysatoren beruht.

Weiterhin ist aus dem Datenblatt der Firma Emery eine Weichwachermischung für Polyamid-Harze mit der Bezeichnung LOXIOL 2472 bekannt.

### Offenbarung der Erfindung

Beim nicht zur Erfindung gehörigen Verfahren zur Herstellung eines Esters aus einer Hydroxybenzoesäure und einem Fettalkohol zur Verwendung in einem Bindersystem für Pulverspritzguss durch Umesterung eines Esters aus der Hydroxybenzoesäure und einem niedrigen Alkohol, bilden der Fettalkohol und der Ester aus der Hydroxybenzoesäure und dem niedrigen Alkohol ein Reaktionsgemisch, wobei der Fettalkohol und der Ester aus der Hydroxybenzoesäure und dem niedrigen Alkohol im Wesentlichen äquimolar eingesetzt werden. Die Umesterung wird bei einer Temperatur durchgeführt, in der der Ester aus der Hydroxybenzoesäure und dem niedrigen Alkohol und der Fettalkohol als homogene Schmelze vorliegen.

"Im Wesentlichen äquimolar" im Sinne der vorliegenden Erfindung bedeutet, dass der Ester aus der Hydroxybenzoesäure und dem niedrigen Alkohol und der Fettalkohol in einem Mol-Verhältnis von maximal 40:60, bevorzugt von maximal 45:55 und insbesondere von maximal 48:52 vorliegen, wobei jede der beiden Komponenten, bevorzugt jedoch der Fettalkohol, im Überschuss vorliegen kann.

Im Unterschied zu Reaktionen der Hydroxybenzoesäure mit Fettalkoholen kann die Umesterung von Estern aus der Hydroxybenzoesäure und niedrigen Alkoholen mit Fettalkoholen bei niedrigeren Temperaturen durchgeführt werden. Aufgrund der niedrigeren Reaktionstemperaturen wird vermieden, dass die Hydroxybenzoesäure zu Phenol decarboxyliert. Auf diese Weise kann die Bildung von Phenol stark reduziert oder sogar vermieden werden. Die Durchführung der Reaktion bei niedrigeren Temperaturen als die der Reaktion einer Hydroxybenzoesäure mit einem Fettalkohol ist darauf zurückzuführen, dass der Ester aus der Hydroxybenzoesäure und einem niedrigen Alkohol und der Fettalkohol bereits bei geringeren Temperaturen eine gemeinsame flüssige Phase bilden. Die Temperatur, bei der beispielsweise p-Hydroxybenzoesäure-Ethylester mit einer äquimolaren Menge an 1-Docosanol eine homogene flüssige Phase bildet, liegt im Allgemeinen bereits bei 100°C. Demgegenüber bildet eine Hydroxybenzoesäure mit einem Fettalkohol erst bei Temperaturen von mehr als 195°C eine homogene flüssige Phase. Bei diesen Temperaturen setzt jedoch bereits die Decarboxylierung der p-Hydroxybenzoesäure ein.

Unter einem Fettalkohol im Sinne der vorliegenden Erfindung wird ein 1-Alkanol mit mindestens 16 C-Atomen verstanden. Typischerweise eingesetzte Fettalkohole sind zum Beispiel 1-Docosanol (C₂₂H₄₅OH), 1-Eicosanol (C₂₀H₄₁OH) oder 1-Octadecanol (C₁₈H₃₇OH) sowie Mischungen daraus. Fettalkohole werden meist aus Naturprodukten wie Bienenwachs gewonnen. Üblicherweise fallen diese als Gemische an.

Im Allgemeinen werden für die Synthese von technischen Produkten, beispielsweise von Estern, die aus Naturprodukten anfallenden Gemische von Fettalkoholen eingesetzt, wobei deren Zusammensetzung schwankend ist. Bei chemischen Umsetzungen dieser Fettalkoholgemische erhält man keine reinen Produkte, sondern Produktgemische. Jedoch kann die Schmelze eines solchen Produktgemisches beim Abkühlen fraktioniert erstarren, so dass die Zusammensetzung des festen Produkts im Gebinde lokal unterschiedlich ist. Dies führt zu Feedstocks schwankender Zusammensetzung und schwankender Eigenschaften, was beim Prozess des Pulverspritzgießens störend ist. Um solche Inhomogenitäten zu vermeiden, ist es daher bevorzugt, dass der zur Herstellung des Esters eingesetzte Fettalkohol mindestens 90 Gew.-% einer definierten Fettalkohol-Komponente enthält. Definierte Fettalkohole mit unterschiedlichen Reinheitsgraden sind im Handel erhältlich.

Unter der Bezeichnung "Hydroxybenzoesäure" wird erfindungsgemäß o-Hydroxybenzoesäure, p-Hydroxybenzoesäure oder ein beliebiges Gemisch aus o-Hydroxybenzoesäure und p-Hydroxybenzoesäure verstanden. Entsprechend ist ein Ester aus der Hydroxybenzoesäure und einem niedrigen Alkohol bzw. der hergestellte Ester aus der Hydroxybenzoesäure und einem Fettalkohol ein Ester der p- Hydroxybenzoesäure, ein Ester der o-Hydroxybenzoesäure oder eine Mischung daraus.

Unter einem niedrigen Alkohol wird erfindungsgemäß ein C₁-C₄-Alkanol verstanden. Bevorzugt bedeutet niedriger Alkohol Methanol, Ethanol, 1-Propanol und 1-Butanol. Besonders bevorzugt als niedrige Alkohole sind Methanol und Ethanol. Die Ester aus der Hydroxybenzoesäure und dem niedrigen Alkohol sind somit insbesondere p-Hydroxybenzoesäuremethylester, o-Hydroxybenzoesäuremethylester, p-Hydroxybenzoesäureethylester und o-Hydroxybenzoesäureethylester. Auch können Mischungen der entsprechenden Ester eingesetzt werden.

Um den Umesterungsgrad bei der Herstellung des Esters aus der Hydroxybenzoesäure und dem Fettalkohol zu erhöhen, ist es bevorzugt, die niedrigen Alkohole, die sich bei der Umesterung abspalten, durch Destillation aus dem Reaktionsgleichgewicht zu entfernen. Aufgrund der bei der Reaktion notwendigen Temperaturen verdampfen die niedrigen Alkohole im Allgemeinen mit ihrer Freisetzung und können so bereits aus dem Reaktionsgemisch als Dampf abgezogen werden. Dies hat weiterhin den Vorteil, dass die niedrigen Alkohole nicht als Verunreinigungen im Produkt, nämlich dem Ester aus der Hydroxybenzoesäure und dem Fettalkohol, enthalten sind.

In einer bevorzugten Ausführungsform wird die Umesterung in Gegenwart eines Katalysators durchgeführt. Als Katalysator eignen sich insbesondere metallorganische, Zinn enthaltende Verbindungen. Durch den Einsatz des Katalysators wird vermieden, dass freie p-Hydroxybenzoesäure bzw. o-Hydroxybenzoesäure als Zwischenprodukt entsteht. Hierdurch kann die unerwünschte Bildung von Phenol durch Decarboxylierung der Hydroxybenzoesäure ausgeschlossen werden.

Ganz besonders bevorzugt ist die als Katalysator eingesetzte metallorganische, Zinn enthaltende Verbindung Dibutyl-Zinnoxid, Dibutyl-Zinndilaurat oder Butyl-Zinnoxid-Hydroxid (Butylstannonsäure). Die eingesetzte Menge an Katalysator, bezogen auf die Masse des Reaktionsgemischs, liegt vorzugsweise im Bereich von 0,1 bis 1 Gew.-%. Besonders bevorzugt beträgt die eingesetzte Menge an Katalysator, bezogen auf die Masse des Reaktionsgemisches, 0,2 bis 0,5 Gew.-%.

Um zu vermeiden, dass die Edukte, d.h. der Ester aus der Hydroxybenzoesäure und dem niedrigen Alkohol und der Fettalkohol oder die Produkte, d.h. der Ester aus der Hydroxybenzoesäure und dem Fettalkohol, oxidieren, und um weiterhin den niedrigen Alkohol aus dem Reaktionsgemisch zu entfernen, wodurch es möglich ist, den eingesetzten Ester aus der Hydroxybenzoesäure und dem niedrigen Alkohol nahezu vollständig zu dem Ester aus der Hydroxybenzoesäure und dem Fettalkohol umzuestern, wird in einer bevorzugten Ausführungsform ein Schutzgasstrom durch die Reaktionsschmelze geleitet, der unter anderem den gebildeten niedrigen Alkohol aus der Schmelze austrägt. Vorzugsweise wird der Schutzgasstrom mit dem niedrigen Alkohol in einen Kondensator geleitet.

Als Schutzgas, das durch das Reaktionsgemisch geleitet wird, eignen sich zum Beispiel Stickstoff, Argon und/oder Kohlendioxid. Davon ist Kohlendioxid besonders bevorzugt, da es der Decarboxylierung von Hydroxybenzoesäuren entgegenwirkt.

Wenn ein Katalysator eingesetzt wird, so erfolgt die Zugabe des Katalysators vorzugsweise portionsweise während des Verlaufs der Umesterung. Mit der Zugabe des Katalysators kann ein geringer Sauerstoff-Eintrag in das Reaktionsgemisch verbunden sein. Um zu vermeiden, dass durch den Eintrag an Sauerstoff Edukte oder Produkte oxidiert werden, ist es bevorzugt, dem Reaktionsgemisch eine geringe Menge an Reduktionsmittel zuzugeben. Der Anteil des Reduktionsmittels, bezogen auf die Masse an Reaktionsgemisch liegt vorzugsweise im Bereich von 0,01 bis 1 Gew.-%. Als Reduktionsmittel eignet sich zum Beispiel Hydrochinon, n-t-Butylphenol oder Benzoin, wobei Hydrochinon besonders geeignet ist, da sich seine braunen Oxidationsprodukte aus einer Lösung der Produkte beispielsweise in Aceton durch Aktivkohle entfernen lassen.

Durch ihre geringen Anteile stören die Reduktionsmittel im Produkt nicht und können bei Bedarf durch Umkristallisieren des Produkts entfernt werden.

Der durch das nicht zur Erfindung gehörige Verfahren hergestellte Ester aus der Hydroxybenzoesäure und einem Fettalkohol ist bei Reaktionstemperatur eine klare farblose Flüssigkeit, die kein freies Phenol enthält. Durch die Reinheit des eingesetzten Fettalkohols, d.h. den Anteil an einer Fettalkohol-Komponente von mehr als 90 Gew.-%, sind die Ester weitgehend einheitlich zusammengesetzt. Es ist keine zusätzliche Reinigung erforderlich, und es bilden sich beim Erstarren keine unterschiedlichen Produktfraktionen. Bei Bedarf ist eine Reinigung von Edukten, Katalysatoren und Reduktionsmittel durch Umkristallisieren in einem geeigneten Lösungsmittel, beispielsweise Aceton, möglich.

Die gebildeten Ester aus der p-Hydroxybenzoesäure bzw. der o-Hydroxybenzoesäure und einem Fettalkohol sind bei einer Temperatur von 48 °C zu mehr als 99 Prozent in Aceton löslich. Lediglich die im Ester enthaltenen Katalysatoren können Rückstände bilden. Zudem kann davon ausgegangen werden, dass die gebildeten Ester weitgehend physiologisch unbedenklich sind.

Die Erfindung betrifft die Verwendung des Esters in einem Bindersystem für ein Pulverspritzgussverfahren, enthaltend 80 bis 98 Gew.-% eines Metallpulvers und/oder eines Keramikpulvers und 1 bis 19 Gew.-% einer polymeren Binderkomponente. Erfindungsgemäß sind weiterhin 1 bis 19 Gew.-% mindestens eines Esters, hergestellt aus Hydroxybenzoesäure und Fettalkohol gemäß dem vorstehend beschriebenen Verfahren, enthalten.

Als polymere Binderkomponente im Bindersystem wird im Allgemeinen ein Polyamid eingesetzt. Geeignete Polyamide sind zum Beispiel Copolyamide 612 (hergestellt aus Caprolactam und Laurinlactam), Mischungen von Polyamid 11 und Polyamid 12 oder Polyether-Blockamide (PEBA).

Üblicherweise für das Pulverspritzgießverfahren eingesetzte Metallpulver sind Pulver, die nur ein Metall oder eine Mischung aus zwei oder mehreren Metallen enthalten und/oder selbst Legierungen darstellen.

Als Keramikpulver können zum Beispiel Pulver aus Aluminiumoxid, Zirkondioxid, Siliciumnitrid oder Siliciumcarbid eingesetzt werden. Auch ist es möglich, dass Mischungen aus zwei oder mehreren der genannten Keramiken eingesetzt werden. Weiterhin ist es ebenfalls möglich, Mischungen zu verwenden, die sowohl Metallpulver als auch Keramikpulver enthalten. Bevorzugt ist es jedoch, Bindersysteme einzusetzen, die entweder Metallpulver oder Keramikpulver enthalten. Wie vorstehend bereits beschrieben handelt es sich bei der Hydroxybenzoesäure, aus der der Ester hergestellt wird, um o-Hydroxybenzoesäure oder p-Hydroxybenzoesäure oder einer Mischung daraus.

Um eine homogene Schmelze zu erhalten, ist es weiterhin bevorzugt, dass der Anteil an einem Ester, der aus einer definierten Fettalkoholkomponente hergestellt ist, mindestens 90 Gew.-%, bezogen auf die Masse des Esters, beträgt.

Das Bindersystem mit dem durch das nicht zur Erfindung gehörige Verfahren hergestellten Ester aus der Hydroxybenzoesäure und einem Fettalkohol als Co-Binder bildet mit einem Polyamid als polymere Binderkomponente eine flüssige Phase mit gemeinsamem Schmelzpunkt. Die gemeinsame Phase aus polymerer Binderkomponente und Co-Binder weist nach dem Erstarren eine hohe Dehnfähigkeit auf. Ein weiterer Vorteil des erfindungsgemäßen Bindersystems ist es, dass der Co-Binder mit einem geeigneten Lösungsmittel, zum Beispiel Aceton, aus der erstarrten gemeinsamen Schmelze extrahierbar ist, ohne dass die verbleibende polymere Binderkomponente, beispielsweise das Polyamid, wesentlich quillt oder rissig wird.

Die Verwendung von Estern aus Hydroxybenzoesäure und Fettalkoholen hat weiterhin den Vorteil, dass die Ester selbst Dispergatoren darstellen und dadurch Metallpulverteilchen bzw. Keramikpulverteilchen in der Schmelze aus polymerer Binderkomponente und Co-Binder in Dispersion gehalten werden und somit ein Zusatz von weiteren Dispergiermitteln, beispielsweise von Fettsäuren, nicht erforderlich ist. Die gemeinsame Phase aus polymerer Binderkomponente und Co-Binder hat weiterhin den Vorteil, dass die Dehnfähigkeit des Compounds ausreichend hoch ist, so dass ein Anguss im Spritzgießwerkzeug bespielsweise als Tunnelanguss ausgeführt werden kann. Weiterhin hat sich gezeigt, dass der nach dem erfindungsgemäßen Verfahren hergestellte Ester aus Hydroxybenzoesäure und Fettalkohol im Spritzgießwerkzeug beim Erstarren nicht ausgeschieden wird und so Poren im gesinterten Gefüge vermieden werden können. Auch weist der Co-Binder einen wachsartigen Charakter auf und wirkt dadurch wie ein Formtrennmittel, wodurch ein Anhaften der spritzgegossenen Bauteile und Angüsse am Werkzeug vermieden werden kann.

## Patentansprüche

1. Verwendung eines Esters aus einer Hydroxybenzoesäure und einem Fettalkohol, in Form eines 1-Alkanols mit mindestens 16 C-Atomen, in einem Bindersystem für ein Pulverspritzgussverfahren, mit einem Anteil von 1 bis 19 Gew.-%, weiter enthaltend 80 bis 98 Gew.-% eines Metallpulvers und/oder eines Keramikpulvers und 1 bis 19 Gew.-% einer polymeren Binderkomponente.

2. Verwendung gemäß Anspruch 1, **dadurch gekennzeichnet, dass** die polymere Binderkomponente ein Polyamid ist.

3. Verwendung gemäß Anspruch 2, **dadurch gekennzeichnet, dass** das Polyamid ein Copolyamid 612, eine Mischung aus Polyamid 11 und Polyamid 12 oder ein Polyether-Blockamid ist.

4. Verwendung gemäß einem der Ansprüche 1 bis 3, **dadurch gekennzeichnet, dass** die Hydroxybenzoesäure o-Hydroxybenzoesäure oder p-Hydroxybenzoesäure ist.

5. Verwendung gemäß einem der Ansprüche 1 bis 4, **dadurch gekennzeichnet, dass** der Anteil an einem Ester, hergestellt aus einer definierten Fettalkoholkomponente, mindestens 90 Gew.-% bezogen auf die Masse des Esters beträgt.

6. Verwendung gemäß Anspruch 1, **dadurch gekennzeichnet, dass** der Fettalkohol 1-Docosanol, 1-Eicosanol, 1-Octadecanol oder Mischungen daraus enthält.

## Claims

1. Use of an ester of a hydroxybenzoic acid and a fatty alcohol in the form of a 1-alkanol having at least 16 carbon atoms in a binder system for a powder injection moulding process, with a proportion of 1% to 19% by weight, further comprising 80% to 98% by weight of a metal powder and/or a ceramic powder and 1% to 19% by weight of a polymeric binder component.

2. Use according to Claim 1, **characterized in that** the polymeric binder component is a polyamide.

3. Use according to Claim 2, **characterized in that** the polyamide is a copolyamide 6/12, a mixture of polyamide 11 and polyamide 12, or a polyether-blockamide.

4. Use according to any of Claims 1 to 3, **characterized in that** the hydroxybenzoic acid is o-hydroxybenzoic acid or p-hydroxybenzoic acid.

5. Use according to any of Claims 1 to 4, **characterized in that** the proportion of an ester prepared from a defined fatty alcohol component is at least 90% by weight, based on the mass of the ester.

6. Use according to Claim 1, **characterized in that** the fatty alcohol comprises 1-docosonal, 1-eicosanol, 1-octadecanol or mixtures thereof.

## Revendications

1. Utilisation d'un ester d'un acide hydroxybenzoïque et d'un alcool gras, sous la forme d'un 1-alcanol contenant au moins 16 atomes C, dans un système de liant pour un procédé de moulage par injection en poudre, en une proportion de 1 à 19 % en poids, contenant en outre 80 à 98 % en poids d'une poudre métallique et/ou d'une poudre céramique et 1 à 19 % en poids d'un composant liant polymère.

2. Utilisation selon la revendication 1, **caractérisée en ce que** le composant liant polymère est un polyamide.

3. Utilisation selon la revendication 2, **caractérisée en ce que** le polyamide est un copolyamide 612, un mélange de polyamide 11 et de polyamide 12 ou un polyéther-co-amide.

4. Utilisation selon l'une quelconque des revendications 1 à 3, **caractérisée en ce que** l'acide hydroxybenzoïque est l'acide o-hydroxybenzoïque ou l'acide p-hydroxybenzoïque.

5. Utilisation selon l'une quelconque des revendications 1 à 4, **caractérisée en ce que** la proportion d'un ester fabriqué à partir d'un composant alcool gras défini est d'au moins 90 % en poids, par rapport à la masse de l'ester.

6. Utilisation selon la revendication 1, **caractérisée en ce que** l'alcool gras contient du 1-docosanol, du 1-eicosanol, du 1-octadécanol ou des mélanges de ceux-ci.
